(19) Europäisches Patentamt
European Patent Office
Office européen des brevets

(11) Veröffentlichungsnummer: **0 281 959 A2**

(12) # EUROPÄISCHE PATENTANMELDUNG

(21) Anmeldenummer: **88103358.3**

(51) Int. Cl.[4]: **C07C 59/315** , C07C 51/58

(22) Anmeldetag: **04.03.88**

(30) Priorität: **07.03.87 DE 3707367**

(43) Veröffentlichungstag der Anmeldung: **14.09.88 Patentblatt 88/37**

(84) Benannte Vertragsstaaten: **BE DE FR GB IT NL**

(71) Anmelder: **HOECHST AKTIENGESELLSCHAFT**
**Postfach 80 03 20**
**D-6230 Frankfurt am Main 80(DE)**

(72) Erfinder: **Schwertfeger, Werner, Dr.**
**Erlenstrasse 8**
**D-6306 Langgöns(DE)**

(54) **Umsetzung von Hexafluorpropenoxid mit fluorierten Carbonsäurefluoriden.**

(57) Verfahren zur Herstellung von halogenierten Carbonsäurefluoriden bei dem ein Carbonsäurefluorid umgesetzt wird mit Hexafluorpropenoxid in Gegenwart eines Katalysatorsystems, das aus einem Alkalifluorid, einem Carbonsäuredinitril und mindestens einem Ether der Formel

$CH_3O-(CH_2CH_2O)_z-CH_3$ (V)

besteht, in der z eine ganze Zahl von 2 bis 6 bedeutet.

EP 0 281 959 A2

## Umsetzung von Hexafluorpropenoxid mit fluorierten Carbonsäurefluoriden

Fluorierte Säurefluoride der allgemeinen Formel

$$R_f-\left[CF_2-O-\overset{CF_3}{\overset{|}{C}}F-\right]_n-COF \qquad (I)$$

in der $R_f$ einen halogenhaltigen, meist fluorhaltigen Rest, vorzugsweise Perfluoralkyl, verzweigt oder unverzweigt mit 1 - 10 Kohlenstoffatomen und n eine ganze Zahl von 1 - 5 bedeutet, werden hergestellt durch Umsetzung von Carbonsäurefluoriden der allgemeinen Formel

$R_f$-COF (II)

in der $R_f$ die obengenannte Bedeutung hat, mit Hexafluorpropenoxid (HFPO). Die Reaktion wird in Gegenwart eines Katalysators durchgeführt und läuft nach folgender Gleichung ab.

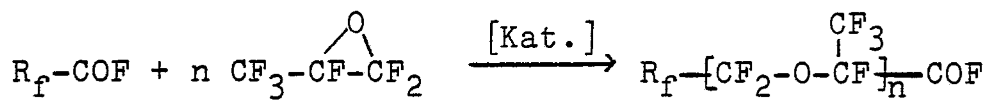


Eine zusammenfassende Darstellung dieser Reaktion einschließlich der verwendeten Katalysatoren ist in Angew. Chem. Int. Ed. Engl. 24, 161-178 (1985) gegeben worden. Einer der dort beschriebenen Katalysatoren ist ein Gemisch aus Kaliumfluorid/Adiponitril und Tetraglyme (Tetraethylenglykoldimethylether). Das Gemisch wurde bisher nur zur Synthese spezieller Verbindungen der allgemeinen Formel (I) eingesetzt, z.B.

a) $CF_3-CF_2-CF_2-O-\overset{CF_2N_3}{\overset{|}{C}}F-\left[CF_2-O-\overset{CF_3}{\overset{|}{C}}F\right]_n-COF$

b) $CH_3OOC-CF_2-\left[CF_2-O-\overset{CF_3}{\overset{|}{C}}F-\right]_n COF$ oder

c) $CH_3-O-CF_2-CF_2-\left[CF_2-O-\overset{CF_3}{\overset{|}{C}}F-\right]_n-COF$ (EP-A-00 70 635, DE-A 31 30 859, EP-A-0 150 618).

Ein Vorteil dieses Katalysatorgemisches soll eine möglichst hohe Ausbeute an gewünschtem Produkt der allgemeinen Formel (I) sein unter gleichzeitiger Vermeidung von Nebenprodukten des Typs

$$CF_3-CF_2-\left[CF_2-O-\overset{CF_3}{\overset{|}{C}}F-\right]_m COF \qquad m = 0 - 5,$$

die durch Reaktion von HFPO mit sich selbst entstehen. Als weiterer Vorteil dieses Katalysatorsystems wird angeführt, daß als Fluoridionenquelle das preisgünstige Kaliumfluorid eingesetzt werden kann.

Die Ausbeuten der beschriebenen Verbindungen der allgemeinen Formel (I) lagen jedoch beim Einsatz

dieses Katalysatorsystems unter 50 %, so daß dessen Verwendung nicht allgemein als vorteilhaft angesehen werden kann.

Die Reaktion von Säurefluoriden der allgemeinen Formel

R - COF (III),

in der R die Gruppe $CCl_3$-oder $X-(CF_2)_p$ ist, wobei X Wasserstoff, Brom oder $SO_2F$ und p eine ganze Zahl von 1 bis 6 ist, mit HFPO wurde bisher nach den Angaben in Gegenwart von Katalysatoren durchgeführt, die das teure Caesiumfluorid enthalten (Angew. Chem. Int. Ed. Engl. 24, 161 (1985)).

Es ergab sich daher die Aufgabe, für diese Umsetzung ein allgemein einsetzbares Katalysatorsystem zu finden, das das teure Caesiumfluorid ersetzen kann und eine gute HFPO-Ausnutzung ermöglicht.

Die Erfindung betrifft ein Verfahren zur Herstellung von halogenierten Carbonsäurefluoriden durch Umsetzung eines Carbonsäurefluorids mit Hexafluorpropenoxid in Gegenwart eines Katalysators, bei dem ein Carbonsäurefluorid der Formel

R-COF (III)

in der R die Gruppe $CCl_3$-oder $X-(CF_2)_n$-ist, wobei X ein Wasserstoffatom, ein Bromatom oder einen Fluorsulfonatrest darstellt und n eine ganze Zahl von 1 bis 6 bedeutet, mit Hexafluorpropenoxid umgesetzt wird in Gegenwart eines Katalysatorsystems, das aus einem Alkalifluorid, einem Carbonsäuredinitril und mindestens einem Ether der Formel

$CH_3O-(CH_2CH_2O)_z-CH_3$ (V)

besteht, in der z eine ganze Zahl von 2 bis 6 bedeutet.

Vorzugsweise wird ein Carbonsäurefluorid der Formel (III) eingesetzt in der R die oben genannte Bedeutung hat und n eine ganze Zahl von 2 bis 4 bedeutet.

Es war überraschend, daß das erfindungsgemäß eingesetzte Katalysatorsystem

- hohe Umsätze des Säurefluorids der allgemeinen Formel (III) bewirkt,
- eine hohe HFPO-Ausnutzung ergibt und
- allgemein einsetzbar ist und dann Ausbeuten ergibt, die deutlich über den Ausbeuten liegen, die bisher bei bestimmten Verbindungen erzielt wurden oder mindestens die Ausbeuten erreicht, die unter Einsatz der Caesiumfluorid-Katalysatoren erreicht werden konnten.

Außerdem ist das Reaktionssystem wesentlich weniger feuchtigkeitsempfindlich im Vergleich zu den anderen bekannten Systemen. Die erzielten Ausbeuten liegen im allgemeinen zwischen 65 und 80 %.

Die nach dem erfindungsgemäßen Verfahren erhaltenen Säurefluoride sind wertvolle Zwischenprodukte auf dem Gebiet der organischen Fluorchemie. Insbesondere werden sie eingesetzt zur Herstellung von Vinylethern der allgemeinen Formel

$$R-CF_2-[O-\overset{\displaystyle CF_3}{\overset{|}{C}}F-CF_2]_q-O-CF=CF_2 \qquad (IV),$$

in der R die obengenannte Bedeutung hat und q eine ganze Zahl von 0 - 2 sein kann.

Das verwendete Katalysatorsystem ist ein Gemisch aus einem Alkalifluorid, einem Carbonsäuredinitril und mindestens einem Ether der Formel (V). Alkalifluoride sind Natriumfluorid und vorzugsweise Kaliumfluorid. Als Carbonsäuredinitril können die Dinitrile von gesättigten aliphatischen Dicarbonsäuren mit 5 bis 8 Kohlenstoffatomen, vorzugsweise Adipinsäuredinitril verwendet werden. Ether der Formel (V) sind Polyethylenglykoldimethylether, vorzugsweise der Dimethylether des Tri-oder Pentaethylenglykols, insbesondere aber die Tetraverbindung (Tetraglyme). Die drei Komponenten des Katalysatorsystems werden im Mengenverhältnis von (5-30):(50-80):(5-40), vorzugsweise (8-20):(55-70):(10-30) Gewichtsprozent, bezogen auf das System, eingesetzt. Es ist zweckmäßig, das Alkalifluorid vor dem Einsatz scharf zu trocknen und fein zu pulverisieren. Das Carbonsäuredinitril der handelsüblichen Reinheit kann ohne weitere Vorbehandlung verwendet werden. Der Ether, z.B. Tetraglyme kann ebenfalls in der angebotenen Reinheit direkt eingesetzt werden. Ferner kann Tetraglyme in Form eines Destillationsschnittes, der aus oligomeren Ethylenglykoldimethylethern mit der Hauptkomponente Tetraglyme besteht, verwendet werden.

Es ist auch nicht nachteilig für die erfindungsgemäße Reaktion, wenn das Tetraglyme eine geringe Menge der Monomethylether-Komponente enthält. So setzt sich z.B. das in Beispiel 1 verwendete Tetraglyme zusammen aus Triglyme 17 Fl.-%, Tetraglyme 65 Fl.-%, Pentaglyme 12 Fl.-%, Hexaglyme 1 Fl.-%, Triglykolmonomethylether 5 Fl.-%, bestimmt durch Gaschromatographie. Das Katalysatorsystem wird in einer Menge von 5 bis 45, vorzugsweise 10 bis 40 Gew.-%, bezogen auf das eingesetzte Carbonsäurefluorid, eingesetzt.

Zur Durchführung der Reaktion gemäß der Erfindung legt man in einem Gefäß, das z.B. aus Glas oder rostfreiem Stahl bestehen kann und das mit einem effektiven Rührer ausgestattet ist, das Katalysator-Gemisch vor und gibt das Säurefluorid der allgemeinen Formel (III) zu. Anschließend gast man die erforderliche Menge an HFPO ein. Es ist von Vorteil, während der gesamten Reaktion kräftig zu rühren. Die Reaktion kann bei Unterdruck, Überdruck oder Normaldruck durchgeführt werden, jedoch ist Normaldruck oder Überdruck, im allgemeinen bis 20 bar, vorzugsweise bis 10 bar, bevorzugt. Die Reaktionstemperatur kann zwischen -30 und +100°C liegen, vorzugsweise wird bei Temperaturen zwischen 0 und 60°C gearbeitet.

**Beispiele**

1) In einen mit einem Rührer ausgestatteten 5-Liter-Glasautoklaven wurden 90 g Kaliumfluorid, 350 ccm Adiponitril und 100 ccm Tetraglyme (weitsiedend) gegeben und in das Gemisch 2050 g $H-CF_2-CF_2-COF$ eingetragen. Anschließend wurde unter gutem Rühren HFPO aufgedrückt, wobei eine exotherme Reaktion einsetzte. Der Druck wurde bei 2-3,5 bar und die Innentemperatur bei 40-50°C gehalten. Insgesamt wurden 2900 g HFPO umgesetzt. Der Ansatz wurde über Nacht bei Raumtemperatur gerührt, wobei der Druck auf weniger als 0,5 bar abfiel. Der Inhalt des Autoklaven wurde einer einfachen Destillation unterworfen. Es wurden 4430 g eines farblosen Destillats mit einem Siedepunkt von 30 - 130°C erhalten, das nach der Gaschromatographie einer mit Methanol veresterten Probe folgende Zusammensetzung aufwies:

| | | Gew.-% | $K_p$ | Ausbeute* |
|---|---|---|---|---|
| $CF_3-CF_2-[CF_2-O-CF(CF_3)]_m-COF$ | m=1 | 11 | 55-58°C | verworfen |
| | m=2 | 1 | 116-120°C | |
| $H-(CF_2)_2-[CF_2-O-CF(CF_3)]_n-COF$ | n=1 | 70 | 72-75°C | 71 % |
| | n=2 | 16 | 126-130°C | 11 % |
| | n=3 | 1 | | |

* bez. auf eingesetztes $H-CF_2-CF_2-COF$

Der Umsatz an $H-CF_2-CF_2-COF$ war größer als 90 %.

2) (Vergleich) In einen mit einem Rührer ausgerüsteten 2-Liter Glasautoklaven wurden ein Katalysatorgemisch aus 30 g Caesiumfluorid und 100 ccm Tetraglyme sowie 880 g $H-CF_2-CF_2-COF$ vorgelegt und wie in Beispiel 1 mit 1975 g HFPO bei 23-29°C und einem Druck von 3,2-3,6 bar umgesetzt. Nach dem Rühren über Nacht war noch ein Restdruck von 1,6 bar vorhanden. Die Aufarbeitung gemäß Beispiel 1 lieferte folgende Ausbeuten:

| | | | | | |
|---|---|---|---|---|---|
| $CF_3-CF_2-[CF_2-O-CF(CF_3)]_m-COF$ | m=1 | Kp | 55-58°C | 520 g | |
| | m=2 | Kp | 116-120°C | 223 g | |
| $H-(CF_2)_2-[CF_2-O-CF(CF_3)]_n-COF$ | n=1 | Kp | 72-75°C | 1067 g | 57 % |
| | n=2 | Kp | 126-130°C | 556 g | 20 % |

Der Umsatz an $H-CF_2-CF_2-COF$ betrug ca. 90 %.

3) In der Apparatur von Beispiel 1 wurden 1416 g Br-$CF_2$-COF, 50 g Kaliumfluorid, 300 ccm Adiponitril und 120 ccm Tetraglyme vorgelegt. Die exotherm verlaufende Reaktion mit 1550 g HFPO erfolgt bei 45-50°C und einem Druck von 2-2,2 bar. Nach dem Ende der HFPO-Zufuhr fiel der Druck rasch auf 0 bar ab. Durch Destillation des Autoklaveninhaltes wurden

2106 g der Verbindung

$$Br-CF_2-[CF_2-O-\underset{}{\overset{CF_3}{\overset{|}{C}}}F-]_nCOF$$

mit n = 1 (= 77 %) Kp 71-76°C und

62 g der Verbindung mit n = 2 (= 1,5 %) Kp 128°C erhalten.

4) In einem Glaskolben, der mit einem Kältekühler (-78°C), Thermometer und einem Gaseinleitungsrohr versehen war, wurden 15 g Kaliumfluorid, 100 ccm Adiponitril und 40 ccm Tetraglyme vorgelegt. Diese Mischung wurde abgekühlt und 788 g Br-$CF_2$-$CF_2$-COF zugegeben, das auf ~ 5°C vorgekühlt worden war. Anschließend wurden bei Normaldruck innerhalb von 415 Minuten 680 g HFPO eingegast und die Innentemperatur des Ansatzes unterhalb von 50°C gehalten. Durch Destillation wurden die folgenden Säurefluoride isoliert:

$$Br-(CF_2)_2-CF_2-O-\overset{CF_3}{\overset{|}{C}}F-COF \quad Kp\ 97-99°C, \quad 1065\ g \quad 78\ \%$$

$$Br-(CF_2)_2-[CF_2-O-\overset{CF_3}{\overset{|}{C}}F-]_2COF \quad Kp\ 147°C, \quad 151\ g \quad 8\ \%$$

5) In einem Glaskolben, ausgestattet wie in Beispiel 4, wurden 90 g Kaliumfluorid, 350 ccm Adipinsäuredinitril und 100 ccm Tetraglyme vorgelegt. Anschließend wurden 2465 g

$$\overline{CF_2-CF_2-O-SO_2}$$

zugetropft, das unter exothermer Reaktion sofort zu $FSO_2$-$CF_2$-COF isomerisierte. Danach wurden 4370 g HFPO eingegast und die Innentemperatur wurde während des Eingasens unter 50°C gehalten. Die Aufarbeitung des Ansatzes erfolgte durch Destillation. Es wurden 3745 g (79 %) $FSO_2$-$CF_2$-$CF_2$-O-$CF(CF_3)$-COF mit einem Siedepunkt von 92-94°C erhalten.

6) In einer Apparatur wie in Beispiel 4 wurden 29 g Kaliumfluorid, 150 ccm Adipinsäuredinitril, 60 ccm Tetraglyme und 1400 g $CCl_3$-COF vorgelegt. Anschließend wurden innerhalb von 4,5 Stunden 1660 g HFPO eingegast, wobei die Innentemperatur durch Kühlung unter 37°C gehalten wurde. Das Reaktionsgemisch wurde einer Destillation unter vermindertem Druck unterworfen, bei der die Innentemperatur unter 120°C gehalten wurde. Rohausbeute 2950 g Säurefluoridgemische. Nach fraktionierter Destillation unter Normaldruck wurden folgende Ausbeuten erhalten:

$$CCl_3-[CF_2-O-\overset{CF_3}{\overset{|}{C}}F-]_nCOF$$

n = 1 Kp 117-121°C, 1924 g 68,6 %

n = 2 Kp 161-163°C, 169 g 4 %

7) In der Apparatur des Beispiels 4 wurden 5 g Kaliumfluorid, 50 ml Adipinsäuredinitril und 20 ml Tetraglyme vorgelegt und 203 g 5-H-Octafluorvaleriansäurefluorid zugetropft. Im Laufe von 40 Minuten wurden 166 g HFPO eingegast und die Innentemperatur durch Eiskühlung bei 25-40°C gehalten. Es wurde eine Stunde bei Raumtemperatur nachgerührt und der Ansatz unter vermindertem Druck destilliert, wobei 317 g Destillat mit einem Siedepunkt von 43-83°C/67 mbar erhalten wurde. In der nachgeschalteten

Kältefalle (-78°C) kondensierten 39 g Substanz. Destillat und Falleninhalt wurden vereinigt. Zur Bestimmung der Zusammensetzung wurde eine Probe mit Methanol verestert. Das Estergemisch zeigte bei der gaschromatographischen Analyse folgende Zusammensetzung:

ca. 4 Fl.-% $CF_3-CF_2-CF_2-O-CF(CF_3)-COOCH_3$

ca. 4 Fl.-% n=0 $H-(CF_2)_4-[CF_2-O-CF(CF_3)]_n-COOCH_3$
ca. 80 Fl.-% n=1
ca. 8 Fl.-% n=2

Durch Destillation über eine Füllkörperkorperkolonne wurden 240.5 g (≙ 71 %) $H-(CF_2)_5-O-CF(CF_3)-COF$, Kp 115-119°C, isoliert.

## Ansprüche

1. Verfahren zur Herstellung von halogenierten Carbonsäurefluoriden durch Umsetzung eines Carbonsäurefluorids mit Hexafluorpropenoxid in Gegenwart eines Katalysators, dadurch gekennzeichnet, daß ein Carbonsäurefluorid der Formel
R-COF (III)
in der R die Gruppe $CCl_3$-oder $X-(CF_2)_n$-ist, wobei X ein Wasserstoffatom, ein Bromatom oder einen Fluorsulfonatrest darstellt und n eine ganze Zahl von 1 bis 6 bedeutet, umgesetzt wird mit Hexafluorpropenoxid in Gegenwart eines Katalysatorsystems, das aus einem Alkalifluorid, einem Carbonsäuredinitril und mindestens einem Ether der Formel
$CH_3O-(CH_2CH_2O)_z-CH_3$ (V)
besteht, in der z eine ganze Zahl von 2 bis 6 bedeutet.

2. Verfahren nach Anspruch 1, dadurch gekennzeichnet, daß als Carbonsäurefluorid eine Verbindung der Formel (III) verwendet wird, in der R die genannte Bedeutung hat, worin n aber 2, 3 oder 4 bedeutet.

3. Verfahren nach Anspruch 1 oder 2, dadurch gekennzeichnet, daß als Alkalifluorid Kaliumfluorid verwendet wird.

4. Verfahren nach einem oder mehreren der Ansprüche 1 bis 3, dadurch gekennzeichnet, daß als Carbonsäuredinitril ein Dinitril einer gesättigten aliphatischen Dicarbonsäure mit 5 bis 8 Kohlenstoffatomen, vorzugsweise Adipinsäuredinitril verwendet wird.

5. Verfahren nach einem oder mehreren der Ansprüche 1 bis 4, dadurch gekennzeichnet, daß als Ether der Formel (2) der Dimethylether von Tri-, Tetra-oder Pentaethylenglykol verwendet wird.

6. Verfahren nach einem oder mehreren der Ansprüche 1 bis 5, dadurch gekennzeichnet, daß die Umsetzung bei einer Temperatur von -30 bis +100°C, vorzugsweise zwischen 0 und 60°C, durchgeführt wird.

7. Verfahren nach einem oder mehreren der Ansprüche 1 bis 7, dadurch gekennzeichnet, daß die Komponenten des Katalysatorsystems in einer Menge von 5 bis 30 Gew.-% Alkalifluorid, 50 bis 80 Gew.-% Carbonsäuredinitril und 5 bis 40 Gew.-% Ether verwendet werden. jeweils bezogen auf das Katalysatorsystem.

8. Verfahren nach Anspruch 7, dadurch gekennzeichnet, daß die Komponenten im Verhältnis (8-20):(55-70):(10-30) eingesetzt werden.

9. Verfahren nach einem oder mehreren der Ansprüche 1 bis 8, dadurch gekennzeichnet, daß das Katalysatorsystem in einer Menge von 5 bis 45 Gew.-%, bezogen auf das Carbonsäurefluorid, verwendet wird.

10. Verfahren nach Anspruch 9, dadurch gekennzeichnet, daß eine Katalysatormenge von 10 bis 40 Gew.-% verwendet wird.